# EUROPEAN PATENT APPLICATION

(11) **EP 1 752 130 A1**
(43) Date of publication of application: **14.02.2007**
(21) Application number: 05746049.5
(22) Date of filing: 03.06.2005
(51) Int. Cl.: A61K 8/18

(54) **HAIR STYLE CONTROL AGENT**

(30) Priority: 03.06.2004 JP 2004166020; 02.06.2005 JP 2005162176
(71) Applicant: KAO CORPORATION, Tokyo 103-8210 (JP)
(72) Inventor: ISHII, Daisuke, c/o Kao Corporation Research Lab., Tokyo 131-8501 (JP); TSUCHIYA, Masaru, c/o Kao Corporation Research Lab, Tokyo 131-8501 (JP)
(74) Representative: HOFFMANN EITLE
(86) International application number: PCT/JP2005/010239
(87) International publication number: WO 2005/117815

(57) **Abstract**

A first hair style control agent containing:
(A) a keratin-reducing substance,
(B) a guanidine derivative represented by the general formula (1): 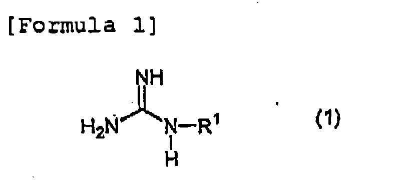 wherein R¹ represents a linear or branched alkyl group having 6 to 12 carbon atoms, or an acid addition salt thereof, and
(C) a nonionic surfactant represented by the general formula (2):

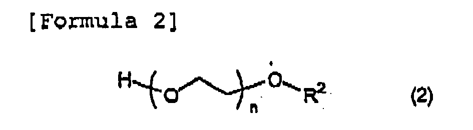

wherein R² represents a linear or branched alkyl group having 8 to 18 carbon atoms, and n represents an integer of 1 to 25. The first hair style control agent of the present invention may permanently straighten naturally curly/unruly hair, without damaging or providing a dry feeling to the hair.

## Description

### Field of the Invention

The present invention relates to a first hair style control agent which contains a guanidine derivative andmaypermanently straighten naturally curly/unruly hair, without damaging or providing a dry feeling to the hair.

### Background of the Invention

Naturally curly/unruly hair often becomes a cause of distress such as looking like the hair is unkempt, the sticking up of hair ends and the lack of beauty in flow of the hair; therefore, various methods for straightening hair have been proposed. Among such straighteningmethods, a representative one is a method called a straight perm, by which hair is reduced enough to cleave the disulfide bonds in hair keratin, straining the hair in a straight manner, for example, by sticking it on a panel or combing it, then fixing the hair in the straightened state, followed by the regeneration of the disulfide bond by oxidation.

In the case where the wave in the hair is caused by a permanent wave treatment, the hair may be sufficiently straightened by a straight perm; however, it is difficult sufficiently to straighten naturally curly/unruly hair by such method. And even if hair seemed apparently straightened, sufficient effect could not be obtained; for example, the original unruliness returned after several days. For this reason, in order to improve the efficiency of the straightening by heat denaturation or further reduction in addition to the conventional straight perm treatment, a method in which hair is heated in the reduced state with an iron (see Patent Document 1), a method in which the reduced hair is steamed and reduced again with a gelatinous first agent (see Patent Document 2), and other similar methods have been proposed. Because hair suffers significant damage if the treatment condition is made severe to heighten the beneficial effect, means such as using sulfite or its analogues which have a lower reducing power, lowering pH of a first agent, combining particular surfactants or oil solutions, or the like have been contrived.

In any of these methods, however, the effect of straightening naturally curling/unruly hair was not fully satisfactory. Hence, a method of combining and using guanidine derivatives or their acid addition salts (see Patent Document 3 and 4) and the like have been proposed as means of solving these problems. In addition, a hair setting agent which contains a guanidine derivative having an amide group and is used without being combined with a reducing composition has been proposed in order to improve the efficiency of the setting of the straightening method (see Patent Document 5).

In the method in which a combination of guanidine derivatives or their acid addition salts is used, however, while the damage to hair caused by a hair keratin reducing agent could be diminished, there was a tendency that the finish of treated hair was dry and coarse because lipid ingredients which were necessary for the retention of water within the hair were extracted by an organic solvent as various organic solvents were used for the purpose of solving various guanidine derivatives or their acid addition salts.

[Patent Document 1] JP-A-60-21704
[Patent Document 2] JP-A-59-90508
[Patent Document 3] JP-A-10-17441
[Patent Document 4] JP-A-10-17442
[Patent Document 5] JP-A-2004-75588

### Disclosure of the Invention

The present invention provides a first hair style control agent containing:
(A) a keratin-reducing substance,
(B) a guanidine derivative represented by the general formula (1):

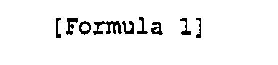

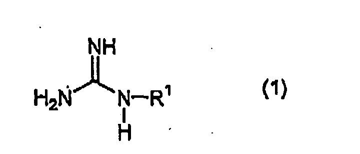

wherein R¹ represents a linear or branched alkyl group having 6 to 12 carbon atoms, or an acid addition salt thereof,
(C) a nonionic surfactant represented by the general formula (2):

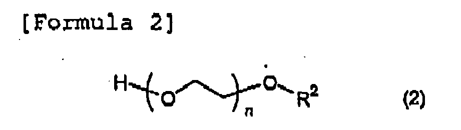

wherein R² represents a linear or branched alkyl group having 8 to 18 carbon atoms, and n represents an integer of 1 to 25.

### Modes for Carrying Out the Invention

The present invention relates to a hair treatment agent composition which can permanently straighten naturally curly/unruly hair, without damaging or providing a dry feeling to the hair.

The present inventors have discovered that a first hair style control agent which is excellent in hair straightening effect, such as permanently straightening naturally curly/unruly hair and permanently setting the hair, without damaging or providing a dry feeling to the hair is obtained by using a keratin-reducing substance, a guanidine derivative represented by the general formula (1) or its acid addition salt, and a particular surfactant in combination.

As the keratin-reducing substance (A), any compound that can reduce keratin, which is a structural protein in hair, can be used. Examples of the ingredient (A) include thioglycolic acid, thioglycolic acid derivatives, salts of thioglycolic acid or thioglycolic acid derivatives, cysteine, salts of cysteine or cysteine derivatives, sulfite, hydrogen sulfite, thioglyceryl alkyl ether represented by the general formula (3):

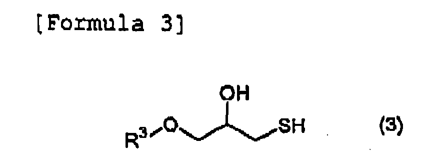

wherein R³ represents a hydrogen atom, a linear or branched alkyl group having 1 to 4 carbon atoms or Rₐ-O-R_{b}, in which Rₐ represents a linear or branched alkyl group having 1 to 4 carbon atoms, and R_{b} represents methylene group or a linear or branched alkylene group having 2 to 4 carbon atoms, or its derivatives and their salts,
and mercaptoalkylamide represented by the general formula (4):

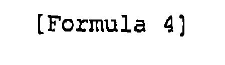

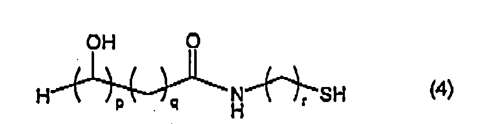

wherein p represents an integer of 0 to 5, q represents an integer of 0 to 3, and r represents an integer of 2 to 5, provided that p and q do not simultaneously represent 0, or its salts.

Among these reducing substances, particular preference is given to thioglycolic acid and its glycerol ester among thioglycolic acids; L-cysteine, D-cysteine, N-acylcysteine and the like among cysteines; ammonium salt, quaternary salt, and alkanol amine salts (monoethanolamine salt, diethanolamine salt, triethanolamine salt and the like) of cysteines among salts of these cysteines; sodium sulfite and the like among sulfites; sodium hydrogensulfite and the like among hydrogensulfites; ethoxy-hydroxypropanethiol, ethoxyethoxy-hydroxypropanethiol, methoxyethoxy-hydroxypropanethiol, isopropoxyethoxy-hydroxypropanethiol, and the like among thioglyceryl alkyl ether; mercaptoethylpropanamide, mercaptoethylgluconamide and the like among mercaptoalkylamides.

The keratin-reducing substance (A) may be used singly or in combination of two or more, and is (are) preferably combined with a content of 0.1 to 20 mass% in a first hair style control agent; and more preferably in the content of 3 to 20 mass%, in view of sufficiently reducing hair without damaging hair or skin.

The guanidine derivative (B) is represented by the general formula (1) referred to above. In the general formula (1), linear or branched alkyl groups having 6 to 12 carbon atoms represented by R¹ include, for example, n-octyl group, 2-ethylhexyl group, decyl group, dodecyl group, and n-octyl group and 2-ethylhexyl group are preferred. A guanidine derivative (1) of the ingredient (B) may form an acid addition salt with any physiologically acceptable acid. An acid to be used in the formation of the acid addition salt may be either an organic acid or an inorganic acid. Such organic acids include, for example, organic carboxylic acids such as acetic acid, propionic acid, butyric acid, isobutyric acid, glycolic acid, lactic acid, succinic acid, malic acid, and the like; lower alkylsulfonic acids such as methanesulfonic acid, isethionic acid, aminoethylsulfonic acid, and the like; arylsulfonic acids such as benzenesulfonic acid, p-toluenesulfonic acid, nitrobenzenesulfonic acid, xylenesulfonic acid, cumenesulfonic acid, and the like. Such inorganic acids include hydrohalic acids such as hydrochloric acid, hydrobromic acid and hydroiodic acid; and further, perchloric acid, sulfuric acid, nitric acid, phosphoric acid, carbonic acid, and the like.

A combination of two or more guanidine derivatives or their acid addition salts may be used as the ingredient (B). The content of guanidine derivatives or their acid addition salts is preferably not less than 1 mass%, more preferably not less than 3 mass%, even more preferably not less than 5 mass% in the first hair style control agent of the present invention from the viewpoint of the straightening effect on curly hair. And, the upper limit of the content is preferably not more than 20 mass%, more preferably 18 mass%, even more preferably 15 mass%.

The nonionic surfactant (C) is polyoxyethylene alkyl ether represented by the general formula (2) referred to above. In the general formula (2), R² represents a linear or branched alkyl group having 8 to 18 carbon atoms, and n which represents an averaged molar number of added ethylene oxide groups is 1 to 25. And n is preferably 2 to 25, more preferably 6 to 23. The ingredient (C) may be a mixture of the compounds in which the numbers of carbon atoms in R² are the same and the values of n are different, or the value of n are the same and the numbers of carbon atoms in R² are different. Two or more of these mixtures may be used in combination. Specific examples of the ingredient (C) may include EMULGEN 102K, EMULGEN 103, EMULGEN 104P, EMULGEN 106, EMULGEN 109P, EMULGEN 3.23P, EMULGEN 150, EMULGEN 1620G, EMULGEN 220 and the like (all manufactured by Kao Corp.), NIKKOLBL-2, NIKKOLBL-9EX, NIKKOLBD-4 and the like (all manufactured by Nikko Chemicals Co. Ltd.).

The content of the nonionic surfactant (C) is preferably 0.1 to 20 mass%, more preferably 3 to 18 mass% in the first hair style control agent of the present invention from the viewpoint of the straightening effect on curly hair.

In the present invention, in the case where an inorganic acid addition salt is used as a guanidine derivative, it is preferable to use polyoxyethylene alkyl ether in which the alkyl chain is relatively short and the molar number of EO addition is small, from the viewpoint of the stability of the composition. On the other hand, in the case where an organic carboxylic acid addition salt, for example, lactate is used as an acid addition salt of a guanidine derivative, it is preferable to use polyoxyethylene alkyl ether in which the alkyl chain is relatively long and the molar number of EO addition is large. For example, for a sulfate or carbonate of a guanidine derivative, it is preferable to use polyoxyethylene alkyl ether in which R² in the general formula (2) is a linear alkyl group having 8 to 12 carbon atoms, more preferably 8 to 10 carbon atoms, and n is 2 to 10, more preferably 6 to 9. And, for the lactate, glycolate, succinate and the like of a guanidine derivative, it is preferable to use polyoxyethylene alkyl ether in which R² in the general formula (2) is an alkyl group having 14 to 18 carbon atoms, more preferably 16 to 18 carbon atoms, and n is 11 to 25, more preferably 16 to 20.

In addition, the ratio by weight of the guanidine derivative of the ingredient (B) and the nonionic surfactant of the ingredient (C) is preferably 5:1 to 1: 5, more preferably 2:1 to 1:1, from the viewpoint of the solubility of guanidine derivatives.

The first hair style control agent of the present invention may contain further glycine betaine (trimethylglycine) as the ingredient (D) from the viewpoint of the straightening effect (wave effect, straightening effect on curly hair) and the sense to the touch. As a glycine betaine, for example, commercially available AMINOCOAT (Asahi Kasei Corp.) may be used. Glycine betaine is preferably contained not less than 0.1 mass%, more preferably not less than 1.5 mass%, even more preferably not less than 2 mass% in the first hair style control agent of the present invention. The upper limit of the content is preferably not more than 12 mass%, more preferably not more than 10 mass%, even more preferably not more than 8 mass%, from the viewpoint of the stability of the composition.

The first hair style control agent of the present invention may be alkalized with an alkaline agent such as ammonia or its salt, alkanolamines and the like, but preferably using ammonia or its salt and monoethanolamine. The pH of the first hair style control agent is adjusted preferably to 5 to 11, more preferably to 6 to 10, even more preferably to 7 to 9.6.

It is also preferable that the hair style control agent of the present invention is made gel-based using a natural polymer or synthetic polymer. Natural polymers and synthetic polymers which may be used herein include dimethyldiallylammonium chloride-acrylamide copolymer [for example, MERQUAT 100, MERQUAT 550 (Calgon Corpoxation)], vinylpyrrolidone-dimethyl aminoethyl methacrylate copolymer [for example, Copolymer 845, Copolymer 937, Copolymer 958 (GAF Corp.)], O-[2-hydzoxy-3-(lauryldimethylammonio)propyl]hydroxyethyl cellulose chloride [for example, Polymer JR-125, JR-30M, JR 400 (Union Carbide Corp.), Leogard G (Lion Corp.)], 0-[2-hydroxy-3-(trimethylammonio)propyl] chloride guar gum (for example, RHABALL gum CG-M, RHABALL gum CG-6L, RHABALL gum CG-M7, RHABALL gum CG-M8M (Dainippon Pharmaceutical Co. Ltd.), Jaguar C-13S, Jaguar C-14S, Jaguar C-17, Jaguar C-210, Jaguar C-162, HI-CARE1000 (allmanufacturedby, Rhone-Poulenc Inc.)], cationized dextran, methyl cellulose [for example, METOLOSE SM (Shin-Etsu Chemical Co., Ltd.), ethyl cellulose [for example, Brilliance 515 (Ikeda Bussan, Ltd.), hydroxyethyl cellulose [for example, CELLOSIZE QP4400H, QP52000H (Union Carbide Corp.), SE-600 (Daicel Chemical Industries, Ltd.)], hydroxypropyl cellulose [for example, Nisso HPC-H, HPC-M (Nippon Soda Co., Ltd.)], hydroxypropylmethyl cellulose [for example, METOLOSE 60SH series, METOLOSE 65SH series, METOLOSE 90SH series (Shin-Etsu Chemical Co., Ltd.), pullulan (for example, pullulan PF-20, pullulan PI-20 (Hayashibara Shoji, Inc.), fatty acid ester of pullulan, xanthan gum, hydroxyethyl xanthan gum [for example RHABALL gum EX (Dainippon Pharmaceutical Co. Ltd.) and the like.

A combination of two or more of these natural polymers and synthetic polymers also may be used. The content of the polymer(s) is preferably 0.05 to 20 mass%, more preferably 0.1 to 10 mass%, even more preferably 0.5 to 5 mass% in the first hair style control agent of the present invention.

The first hair style control agent of the present invention may contain an organic solvent. The organic solvents include monohydric alcohols such as ethanol; monohydric or polyhydric aromatic alcohols such as 2-phenoxyethanol, benzyl alcohol, 2-phenylethanol, and 2-benzyloxyethanol; glycols such as triethylene glycol, 1,3-butylene glycol, propylene glycol, hexylene glycol, and diethylene glycol; glycol ethers such as ethylene glycol methyl ether, ethylene glycol ethyl ether, ethylene glycol mono n-butyl ether, propylene glycol monomethyl ether, diethylene glycol monomethyl ether, diethylene glycol ethyl ether, and diethylene glycol mono n-butyl ether; N-alkylpyrrolidone such as N-methylpyrrolidone and N-ethylpyrrolidone; alkylene carbonate such as ethylene carbonate and propylene carbonate; and lactones such as γ-butyrolactone, γ-valerolactone, and γ-caprolactone.

A combination of two or more of these organic solvents may be used. The content of the solvent (s) is preferably 0.05 to 20 mass%, more preferably 0.1 to 10 mass%, even more preferably 0.5 to 5 mass% in the first hair style control agent of the present invention.

Besides the above-mentioned ingredients, ingredients used in conventional first agents of perm liquids or ingredients used in a cosmetic field may be added to the first hair style control agent of the present invention, depending on the purpose. Such optional ingredients include inorganic alkalis, fatty acids, fats and oils, hydrocarbons, silicone derivatives, surfactants other than the ingredient (C), amino acid derivatives, protein derivatives, chelating agents, preservatives, antioxidants, plant extracts, vitamins, ultraviolet ray absorbents, pH adjusting agents, perfumes and the like.

The impartment of a curly shape or the straightening of curly hair using the first hair style control agent of the present invention can be performed in the same way as conventional perming (straightening, waving, ironing). For instance, in the case of a wave perm, hair is wound on a curler or a rod, impregnated with the first agent and allowed to stand for a prescribed time in the process of treatment with the first agent, followed by the second agent treatment process in which hair is impregnated with the second agent containing an oxidant and allowed to stand for a prescribed time, then hair is detached from the curler or rod, washed with water and dried. Further, between the first treatment process and the second treatment process, hair may undergo an intermediate treatment process in which hair is washed with water or rinsed with an acidic treatment liquid.

In the case of a straight perm in which unruly hair is straightened to become straight hair, unruly hair is set to a straight shape, while being applied with the first agent using a comb or a brush and allowed to stand for a prescribed time in the first agent treatment process, followed by the second agent treatment process in which also hair is applied with the second agent using a comb or brush. Further, between the first treatment process and the second treatment process, hair may undergo an intermediate treatment process in which hair is washed with water or rinsed with an acidic treatment liquid. After a prescribed time for the second agent treatment process, hair is washed with water and dried.

The amount of application of the first hair style control agent of the present invention is preferably 0.1 to 4 times by mass, more preferably 0.5 to 2.5 times by mass of hair to be treated. The time of treatment of hair with the first hair style control agent of the present invention is appropriately adjusted within the range of 1 to 120 minutes, depending upon the degree of unruliness, thickness and the degree of damage of the hair to be treated. In this treatment, hair may be warmed, if necessary, preferably to 30 to 55°C, more preferably to 35 to 50°C.

In the straightening treatment of unruly hair, it is possible to obtain a higher straightening effect by use of a flat-iron. In the case of using a flat-iron, after the first agent treatment process and before the second agent treatment process, the first agent is washed out from the hair, and hair is dried with a dryer or the like and then treated with an iron. It is preferable to set the temperature of the iron to 120 to 180°C, more preferably to 140 to 180°C.

The second agent which is used following the first hair style control agent of the present invention contains an oxidant. The oxidants include bromates, perborates, and hydrogen peroxide; the bromates and perborates include sodium salts, potassium salts and ammonium salts. In the case that the oxidant is hydrogen peroxide, the content of the oxidant is preferably 0. 5 to 3 mass%, more preferably 0.75 to 2.5 mass% in the second agent from the viewpoint of the straightening effect on curly hair and the prevention of damage to hair. An ingredient(s) used in conventional second agents or an ingredient (s) used in a cosmetic field may be added depending upon the purpose. The time of leaving to stand in the treatment is appropriately adjusted between 1 to 30 minutes. Further, besides the first agent of the present invention and the second agent containing an oxidant, a treatment agent which contains an extra hair protection ingredient or the like may be used in combination.

### [Examples]

### Test Examples (solubility score)

First hair style control agents (test solutions) shown in Table 1 were prepared. These test solutions were warmed to 60°C, vigorously stirred and then allowed to stand at room temperature for 12 hours; and the state of dissolution was evaluated by visual observation according to the criteria below.

5: transparent homogeneous solution
4: homogeneous dispersion
3: liquid phase separation
2: precipitation of a small amount of a solid phase
1: precipitation of a solid phase

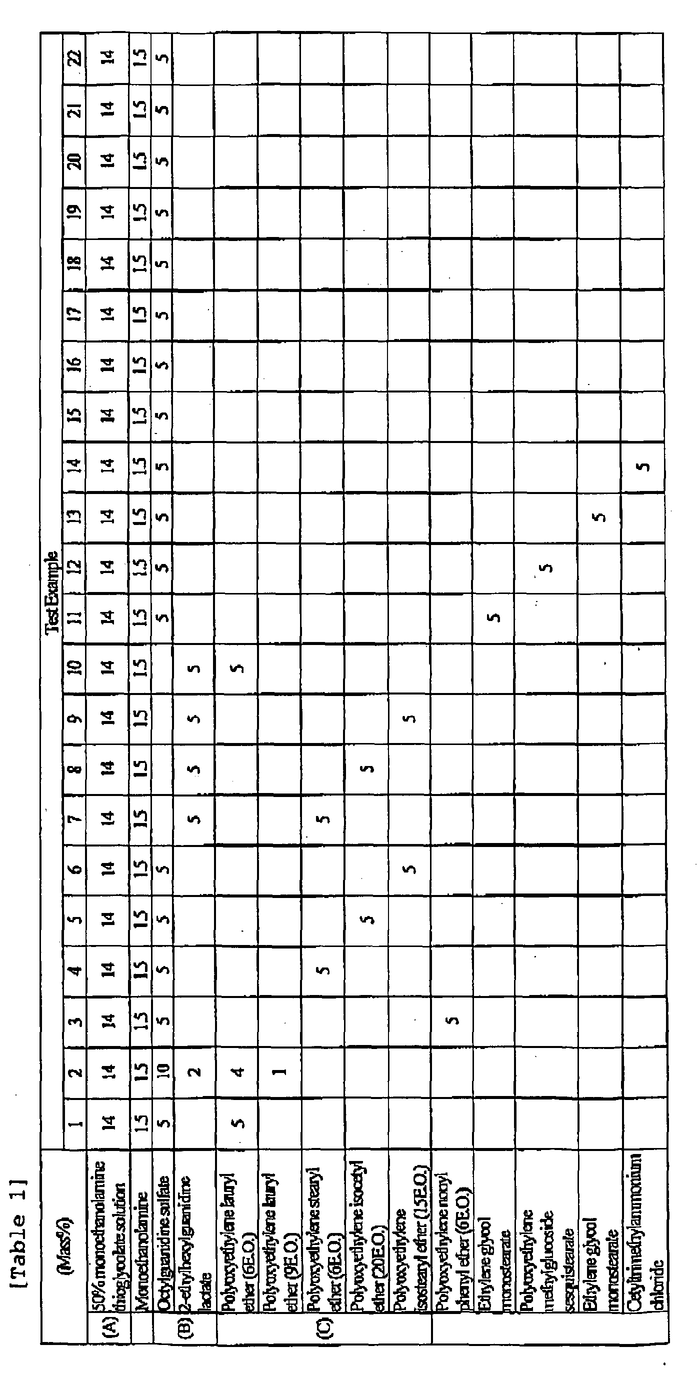

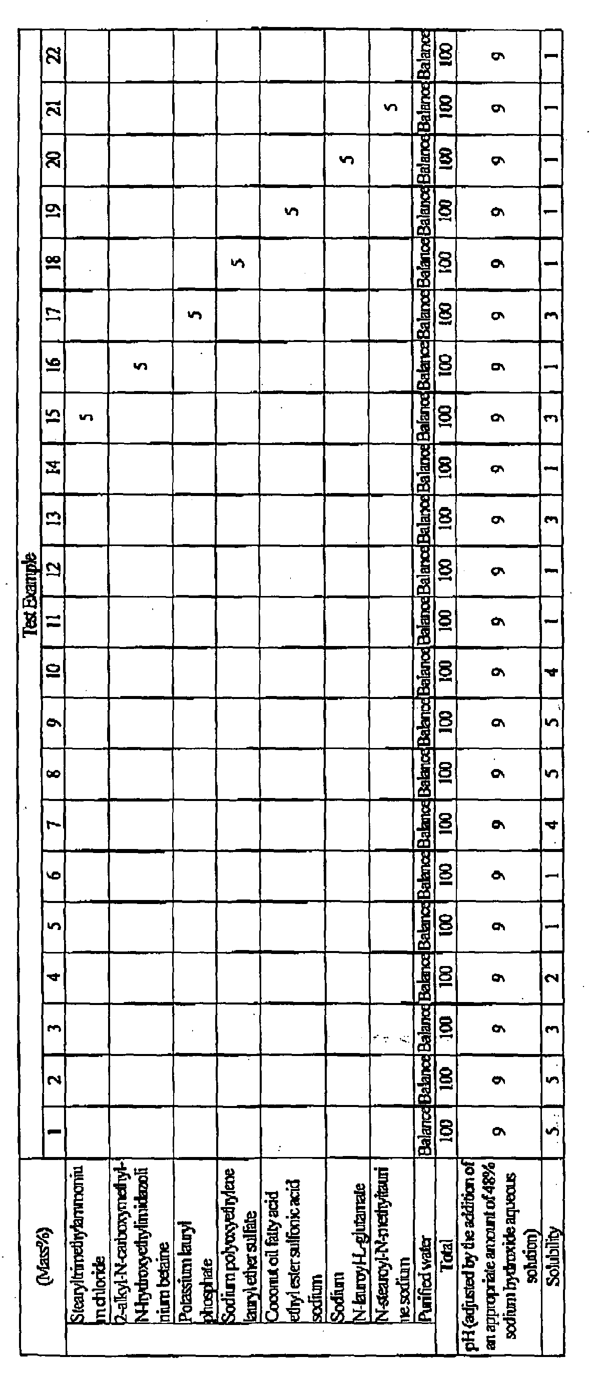

### Examples 1-8 and Comparative Examples 1-9

First agents shown in Table 2 and a second agent shown in Table 3 were prepared. The effect of straightening of curly hair and, the dryness of treated hair when hair was treated using these agents were compared.

### (The effect on undamaged hair)

A tress was prepared using unruly hair with a curl radius of about 5 to 8 mmφ which had not been subjected to chemical treatment such as perming or hair coloring. The first agent is applied to this tress and the tress is allowed to stand for an hour, and then washed with water. To this, the second agent is applied and the tress is allowed to stand for 20 minutes, then washed with water and dried. The straightening effect on curly hair and, dryness were evaluated according to the criteria below.

### (Score of the straightening effect 1)

3: high straightening effect (not less than 25 mmφ)
2: weak straightening effect (not less than 15 mmφ and less than 25 mmφ)
1: hardly reduced unruliness (less than 15 mmφ)

### (Score of dryness)

Treated hairs of each Example and each Reference Example were scored by 5 panelists according to the criteria by which the score of the treated hair of the Comparative Example 9 is 1 and the score of untreated hair is 4. The average of the scores is shown.

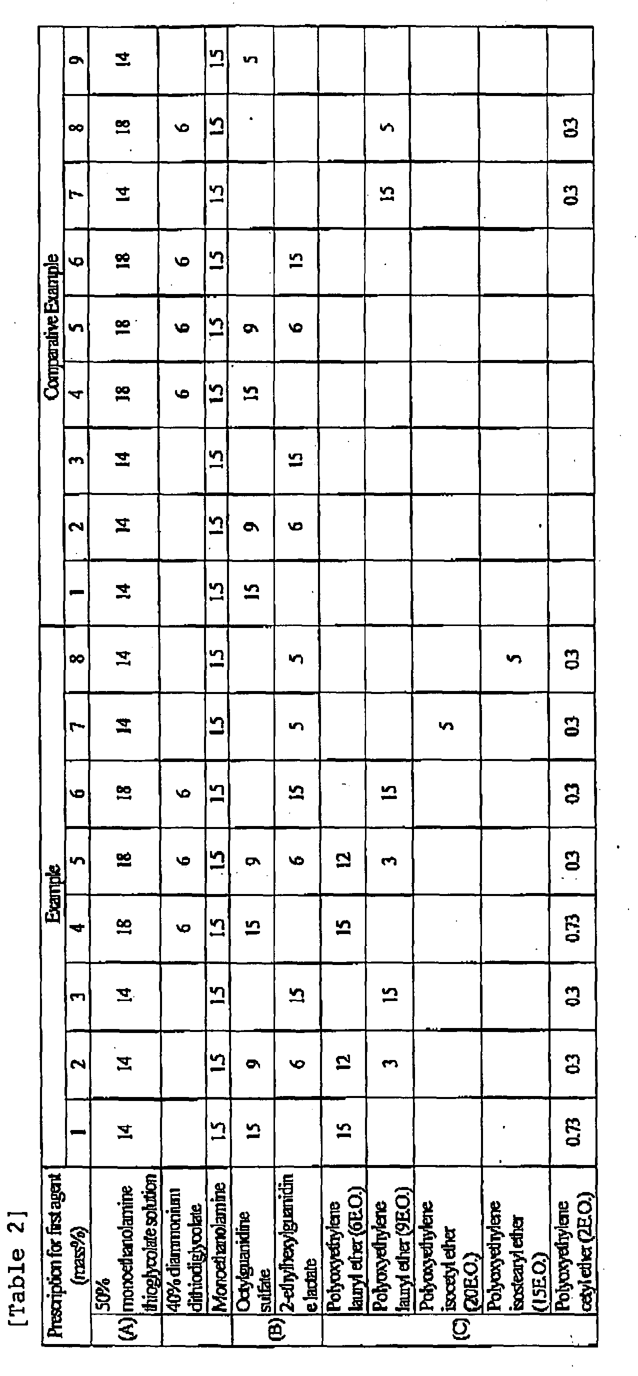

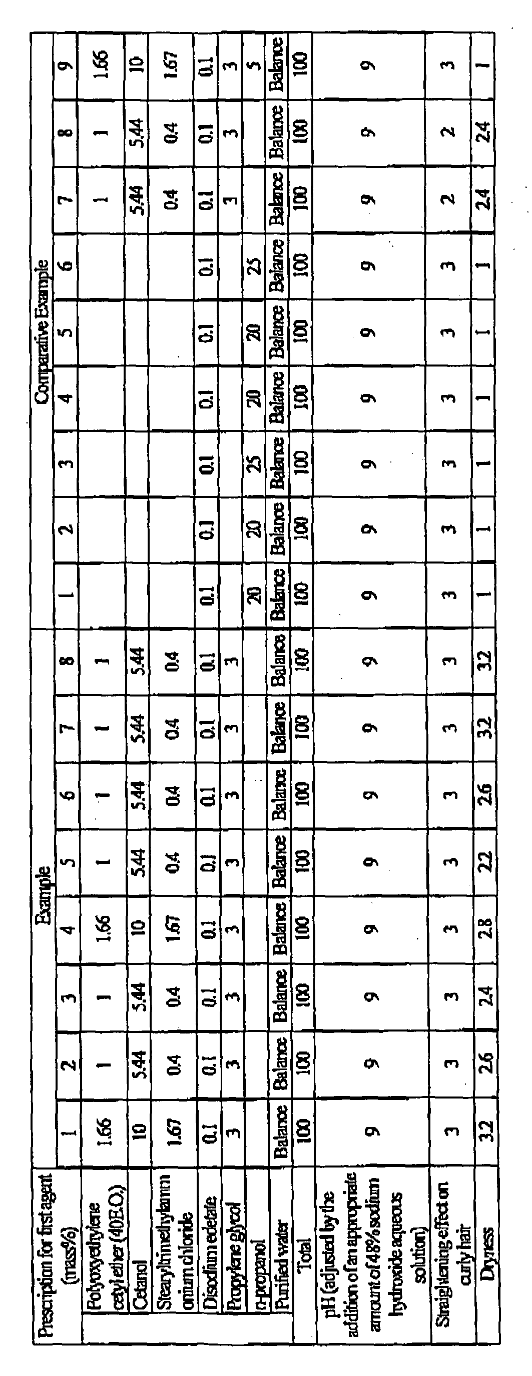

**[Table 3]**

| Prescription for second agent (mass%) | |
|---|---|
| Sodium bromate | 7.20 |
| Cetanol | 7.20 |
| Stearyltrimethylammonium chloride (28%) | 12.90 |
| Polyoxyethylene cetyl ether (2E.O.) | 1.80 |
| Polyoxyethylene cetyl ether (40E.O.) | 1.80 |
| Isopropyl palmitate | 1.80 |
| Polyoxyethylene-methylpolysiloxane copolymer* | 1.00 |
| Aminoethylaminopropylsiloxane-dimethylsiloxa ne copolymer emulsion** | 2.00 |
| Propylene glycol | 3.60 |
| Methyl p-oxybenzoate | 0.10 |
| 75% phosphoric acid aqueous solution | 0.70 |
| 48% sodium hydroxide | Appropriate amount |
| Purified water | Balance |
| Total | 100.00 |
| pH (adjusted by the addition of an appropriate amount of 48% sodium hydroxide aqueous solution) | pH7.0 |

| | |
|---|---|
| *: silicone KF-6005 (Shin-Etsu Chemical Co., Ltd.) **: amino-modified silicone emulsion SM8704C (Shin-Etsu Chemical Co., Ltd.) | |

All of the Examples 1 to 8 which contained the ingredients (A) to (C) gave satisfactory results. Comparative Examples 1 to 6 which used propanol to solve the guanidine derivatives showed dryness. Comparative Examples 7 and 8 which did not contain a guanidine derivative provided an unsatisfactory straightening effect on curly hair.

### Examples 9 to 12

The straightening effects for curly hair and, the dryness of treated hairs were compared in the case where hairs were treated using first agents shown in Table 5 and the second agent shown in Table 3.

### (The effect on undamaged hair)

The straightening effect on undamaged curly hair and, the dryness after treatment was evaluated in the same way as in Examples 1 to 7 and Comparative Examples 1 to 9.

### (The effect on damaged hair)

A bleaching agent prepared by mixing the first agent and the second agent shown in Table 4 with a ratio 1:1 by weight, was applied to a tress of untreated hair similar to those used in the above-described tests, in an amount of equal weight to the tress. The tress was allowed to stand for 20 minutes, then rinsed and dried. Damaged hair was prepared by repeating the above process 21 times.
This damaged hair was subjected to the same treatment as the treatment to undamaged hair. The straightening effect on damaged hair was evaluated according to the criteria below.

### (Score of the straightening effect 2)

3: high straightening effect (not less than 25 mmφ)
2: weak straightening effect (not less than 15 mmφ and less than 25 mmφ)
1: hardly reduced unruliness (not less than 8 mmφ and less than 15 mmφ)
0: hardly reduced or even enhanced unruliness (less than 8 mmφ)

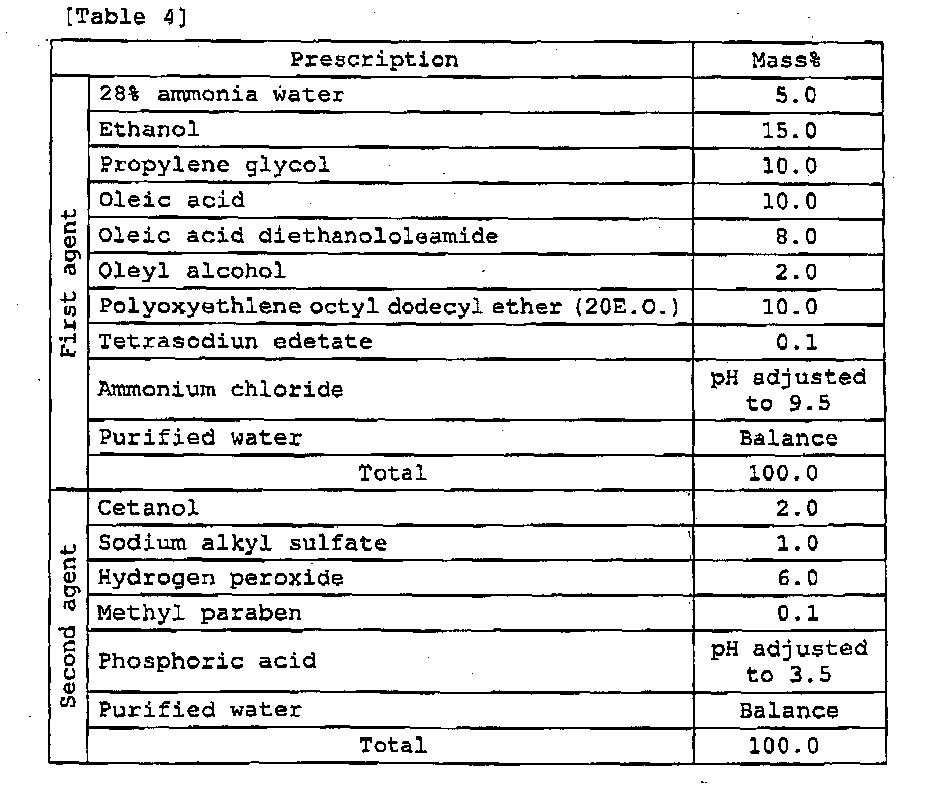

**[Table 5]**

| Prescription for first agent (mass%) | | Example | | | | Comparative Example |
|---|---|---|---|---|---|---|
| | | 9 | 10 | 11 | 12 | 9 |
| (A) | 50 % monoethanolamine thioglycolate solution | 14 | 14 | 18 | 18 | 14 |
| | 40% diammonium dithiodiglycolate | | | 6 | 6 | |
| | Monoethanolamine | 1.5 | 1.5 | 1.5 | 1.5 | 1.5 |
| (B) | Octylguanidine sulfate | 15 | | 15 | | 5 |
| | 2-ethylhexylguanidine lactate | | 5 | | 5 | |
| (C) | Polyoxyethylene lauryl ether (6E.O.) | 15 | | 15 | | |
| | Polyoxyethylene isocetyl ether (20E.O.) | | 5 | | | |
| | Polyoxyethylene isostearyl ether (15E.O.) | | | | 5 | |
| | Polyoxyethylene cetyl ether (2E.O.) | 0.73 | 0.3 | 0.73 | 0.3 | |
| (D) | Glycine betaine | 3 | 3 | 3 | 3 | |
| | Polyoxyethylene cetyl ether (40E.O.) | 1.66 | 1 | 1.66 | 1 | 1.66 |
| | Cetanol | 10 | 5.44 | 10 | 5.44 | 10 |
| | Stearyltrimethylammonium chloride | 1.67 | 0.4 | 1.67 | 0.4 | 1.67 |
| | Disodium edetate | 0.1 | 0.1 | 0.1 | 0.1 | 0.1 |
| | Propylene glycol | 3 | 3 | 3 | 3 | 3 |
| | n-propanol | | | | | 5 |
| | Purified water | Balance | Balance | Balance | Balance | Balance |
| Total | | 100 | 100 | 100 | 100 | 100 |
| pH (adjusted by the addition of an appropriate amount of 48% sodium hydroxide aqueous solution) | | 9 | 9 | 9 | 9 | 9 |
| Straightening effect on curly hair in untreated hair | | 3 | 3 | 3 | 3 | 3 |
| Dryness | | 3.6 | 3.8 | 3.4 | 3.6 | 1 |
| Straightening effect on curly hair in damaged hair | | 3 | 3 | 3 | 3 | 2 |

## Claims

1. A first hair style control agent comprising:
(A) a keratin-reducing substance,
(B) a guanidine derivative represented by the general formula (1): 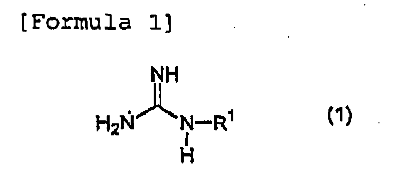 wherein R¹ represents a linear or branched alkyl group having 6 to 12 carbon atoms, or an acid addition salt thereof,and
(C) a nonionic surfactant represented by the general formula (2): 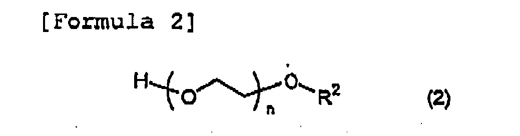
wherein R² represents a linear or branched alkyl group having 8 to 18 carbon atoms, and n represents a number of 1 to 25.

2. The first hair style control agent according to claim 1, further comprising glycine betaine (D).

3. The first hair style control agent according to claim 2, comprising 0.1 to 12 mass% of the ingredient (D).

4. The first hair style control agent according to any of claims 1 to 3, which is used for straightening unruly hair.

5. A method of straightening curly hair comprising using the first hair style control agent according to any of claims 1 to 4 and a flat iron in combination.
